# EUROPÄISCHE PATENTSCHRIFT

(11) **EP 0 347 796 B1**
(45) Veröffentlichungstag und Bekanntmachung des Hinweises auf die Patenterteilung: **04.08.1993**
(21) Anmeldenummer: 89111089.2
(22) Anmeldetag: 19.06.1989
(51) Int. Cl.: C07C 209/36, C07C 217/84

(54) **Verfahren zur Herstellung von 4-Chlor-2,5-dimethoxyanilin**
Preparation process of 4-chloro-2,5-dimethoxy-aniline
Procédé de préparation du 4-chloro-2,5-diméthoxy-aniline

(30) Priorität: 22.06.1988 DE 3821013
(43) Veröffentlichungstag der Anmeldung: 27.12.1989
(73) Patentinhaber: HOECHST AKTIENGESELLSCHAFT, 65926 Frankfurt am Main (DE)
(72) Erfinder: Warning, Klaus, Dr., D-6239 Eppstein/Taunus (DE); Habig, Kurt, Dr., D-6082 Mörfelden-Walldorf (DE)

(56) Entgegenhaltungen:
- DE-A- 2 156 051
- DE-A- 2 308 105
- DE-A- 2 521 303
- FR-A- 2 291 969
- US-A- 3 145 231

## Beschreibung

Die Erfindung betrifft in gegenüber dem Stand der Technik (DE-A 2 521 303 und DE-PS 2 156 051) verbessertes Verfahren zur Herstellung von 4-chlor-2,5-dimethoxyanilin durch katalytische Reduktion von 4-chlor-2,5-dimethoxy-1-nitrobenzol.

Die DE-A-2 521 303 betrifft ein Verfahren zur Herstellung von 2,5-Dimethoxy-4-chloraminobenzol durch katalytische Hydrierung von 2,5-Dimethoxy-4-chlornitrobenzol in Gegenwart unmodifizierter Katalysatoren wie Palladium oder Raney-Nickel und unter Zusatz eines aliphatischen, aromatischen oder heterocyclischen Amins. Das Verfahren läßt allerdings sowohl hinsichtlich Reinheit als auch hinsichtlich der erzielten Ausbeute des Werproduktes noch Wünsche offen.

Bei dem in der vorstehend zitierten deutschen Patentschrift (DE-PS 2 156 051) beschriebenen Verfahren erfolgt die katalytische Reduktion des 4-Chlor-2,5-dimethoxy-1-nitrobenzols in einem aromatischen Lösungsmittel, beispielsweise Xylol, in Gegenwart eines modifizierten Platin auf-KohlenstoffKatalysators, in einem Temperatur- und Druckbereich von etwa 80 bis etwa 110°C bzw. etwa 5 bis etwa 50 atü unter Zusatz von Puffersubstanzen, die in wäßriger Lösung einen pH-Bereich von 8 bis 10 anzeigen. Um hierbei eine normgerechte Base von guter Reinheit, mit hellem Aspekt und in guter Ausbeute zu erhalten, muß der Katalysator jeweils unter Stickstoff abfiltriert und das xylolische Filtrat zwecks Abscheidung der Base kalt gerührt werden (eine vorherige Entfernung des Lösemittels und nachfolgende Abscheidung der Base aus Wasser führt nur zu dunklen und qualitativ unbefriedigenden Produkten; siehe hierzu Vergleichsbeispiel 2). Das nahezu farblos auskristallisierte 4-Chlor-2,5-dimethoxyanilin wird dann durch Filtration isoliert und getrocknet. Das sich bei der Filtration rot färbende Filtrat kann dabei mehrmals in die Reduktion eingesetzt werden, bevor eine destillative Reinigung erfolgen muß.

Nachteilig bei dieser Verfahrensweise ist, daß die Filtration der Base aus dem xylolischen Filtrat mit Lösungsmittelverlusten verbunden ist, die zu unerwünschten Abluftproblemen führen. Außerdem muß das zunächst anfallende xylolfeuchte Basenprodukt noch von anhaftendem Lösungsmittel befreit werden, was eine zusätzliche Operation, entweder in Form einer Trocknung (thermisch) oder einer Behandlung einer wäßrigen xylolfeuchten Basensuspension mit Wasserdampf, bedeutet.

Es bestand daher die Aufgabe, die Reduktion so selektiv zu führen, daß bereits nach beendeter Reduktion eine normgerechte Base vorliegt, die zur Reindarstellung keiner Kristallisation aus der xylolischen Lösung mehr bedarf, sondern welche direkt aus Wasser, und zwar nach Abdestillation des aromatischen Lösungsmittels, beispielsweise Xylol, unter Vakuum oder nach Abblasen des aromatischen Lösungsmittels (Xylols) mit Dampf als normgerechtes Granulat in praktisch quantitativer Ausbeute erhalten werden kann.

Diese Aufgabe wird erfindungsgemäß dadurch gelöst, daß man dem Reduktionsansatz ein geeignetes Amin in katalytischen Mengen zusetzt und im übrigen gemäß dem Verfahren der zitierten DE-PS 2 156 051 verfährt.

Gegenstand der vorliegenden Erfindung ist somit ein Verfahren zur Herstellung von 4-Chlor-2,5-dimethoxyanilin durch katalytische Reduktion von 4-chlor-2,5-dimethoxynitrobenzol mit Wasserstoff in flüssiger Phase bei erhöhter Temperatur und erhöhtem Druck, indem man die Reduktion bei Temperaturen von etwa 80 bis etwa 110°C und einem Druck von etwa 5 bis etwa 50 atü, vorzugsweise etwa 10 bis etwa 20 atü in einem aromatischen Lösungsmittel in Gegenwart eines modifizierten Platin-auf-Kohlenstoff-Katalysators, beispielsweise eines sulfitierten oder sulfidierten, vorzugsweise eines sulfitierten Platin-auf-KohlenstoffKatalysators, in Gegenwart von Wasser und von etwa 0,01 bis etwa 0,2 Mol einer Verbindung, die in wäßriger Lösung einen pH-Wert von 8 bis 10 ergibt, und in Gegenwart von etwa 0,1 bis etwa 1,0 Gew.-%, vorzugsweise etwa 0,2 bis etwa 0,5 Gew.-% eines aliphatischen offenkettigen primären, sekundären oder tertiären Amins oder eines cyclischen Amins, jeweils bezogen auf das eingesetzte 4-Chlor-2,5-dimethoxynitrobenzol, durchführt.

An geeigneten aliphatischen offenkettigen Aminen seien beispielsweise genannt solche der allgemeinen Formeln 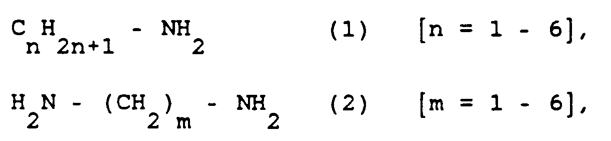
beispielsweise das Ethylendiamin oder das Tetramethylendiamin, wobei das Tetramethylendiamin besonders wirksam ist, 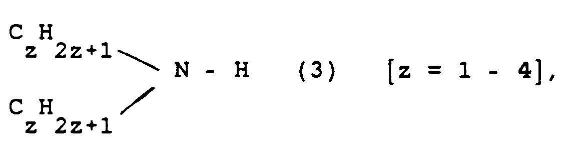
beispielsweise das Di-n-propylamin, und 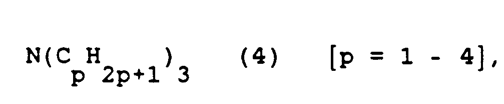
beispielsweise das Tributylamin.

Als geeignete aliphatische cyclische Amine seien beispielsweise Piperidin, Piperazin und insbesondere das Morpholin genannt.

Es können auch Mischungen aus vorstehend genannten Aminen eingesetzt werden.

An geeigneten Verbindungen, die in wäßriger Lösung einen pH-wert von 8 bis 10 ergeben, seien beispielsweise folgende genannt:
Dinatriumdiborat, Borax, Natriumformiat, Natriumacetat, Natriumcarbonat, Dinatriumhydrogenphosphat oder Natriumhydroxid. Anstelle der vorstehend beispielsweise genannten Natriumverbindungen können auch die entsprechenden anderen Alkalimetallverbindungen, teilweise auch die Erdalkalimetallverbindungen, angewandt werden. Es können auch Mischungen aus vorstehend genannten Verbindungen verwendet werden.

Sofern das erfindungsgemäß eingesetzte Amin ausreichend stark basisch ist, kann anstelle einer anorganischen alkalisch wirkenden Verbindung auch das eingesetzte Amin zur Neutralisierung des in geringer Menge (etwa 0,1 Gew.-%) bei der Reduktion freiwerdenden Chlorwasserstoffs bzw. der daraus entstandenen Salzsäure dienen. In diesem Fall kann es erforderlich sein, das Amin in einer höheren Konzentration als weiter oben angegeben einzusetzen.

An aromatischen Lösungsmitteln kommen beispielsweise Benzol, Chlorbenzol, Di- und Trichlorbenzole bzw. deren Gemische, Äthylbenzol, Cumol, vorzugsweise Toluol und insbesondere Xylole in Betracht.

Nach beendeter Reduktion und nach Abfiltration des Katalysators fällt ein organisches Filtrat an, aus dem sich nach Wasserzusatz und nach Entfernung des aromatischen Lösungsmittels (beispielsweise Xylol) (Destillation unter schwachem Vakuum oder unter Normaldruck mit Dampf) überraschenderweise beim Abkühlen die Base als nahezu farbloses, stabiles Granulat in praktisch quantitativer Ausbeute abscheidet.

Es ist bekannt, daß die katalytische Reduktion von halogenhaltigen Nitroaromaten in Gegenwart von aktiven, d. h. nichtsulfidierten bzw. nichtsulfitierten Platin-auf-Kohle-Katalysatoren und mit Morpholin bzw. N-haltigen Basen die Dehalogenierung bei einer Reihe von Chlornitrobenzolen bzw. -toluolen stark verringert (0,1 bis 0,5 %), wobei aber Chlornitroaromaten mit Alkoxygruppen nicht erwähnt werden (US-PS 3 145 231, Annals New York Academy of Sciences 1986, Seiten 175 - 184). In der DE-OS 2 308 105 (Vergleichsbeispiel 2) wurde dagegen festgestellt, daß die Hydrierung von 3,4-Dichlornitrobenzol mit 5 % Pt/Kohle-Katalysator und in Gegenwart von Morpholin zu einer Chlorabspaltung von 1 % und zusätzlich zu einer unbekannten Substanz von 4 Gew.-% führt. Der letzte Befund läßt sich in etwa auch auf die Reduktion des Chlornitroresorcindimethylethers übertragen. Unter vergleichbaren Reaktionsbedingungen beträgt nämlich hier die Chlorabspaltung 1,5 %, was bekanntlich zur unerwünschten Aminohydrochinondimethyletherbildung führt; außerdem werden weitere Nebenprodukte gebildet (z.B. Phenolbildung durch Ethergruppenspaltung), welche der Base einen intensiven blauvioletten Aspekt verleihen (siehe Vergleichsbeispiel 1).

Es ist daher völlig überraschend, daß Morpholin bzw. die anderen verfahrensgemäß eingesetzten Amine, hier in Form eines "Co-Katalysators" einem bereits modifizierten sulfidierten oder sulfitierten Pt/KohleKatalysator zugesetzt, zum einen die Halogenabspaltung auf ≦ 0,2 % verringert und zum anderen vor allem die Bildung der stark färbenden Nebenprodukte verhindert, so daß die Abscheidung des Chloraminohydrochinondimethylethers direkt aus Wasser in technisch besonders eleganter Weise möglich wird.

Für die Durchführung der Reduktion gelten ansonsten im wesentlichen die gleichen Reaktionsbedingungen, wie sie in der DE-PS 2 156 051, Spalte 2, näher beschrieben sind.

Die Reduktion wird so durchgeführt, daß man Nitroverbindung, aromatisches Lösungsmittel, Katalysator, Co-Katalysator und die wäßrige alkalische Lösung in den Autoklav einfüllt und nach Verdrängen der Luft mit Stickstoff unter Rühren aufheizt. Man drückt so lange Wasserstoff auf, bis kein Druckabfall mehr erfolgt. Die gewünschte Reaktionstemperatur wird durch Kühlung von außen aufrechterhalten. Nach Beendigung der Reduktion wird unter Stickstoff der Katalysator abfiltriert, das Lösungsmittel unter Wasserzusatz mit Dampf oder unter schwachem Vakuum abdestilliert und die Base durch Kaltrühren aus Wasser in Granulatform abgeschieden. Diese wird dann als nahezu farbloses Produkt abfiltriert und gegebenenfalls getrocknet. Eine sehr hohe Rückführungszahl des Katalysators ist auch bei dieser Verfahrensweise gewährleistet.

Durch das erfindungsgemäße Verfahren kann das 4-Chlor-2,5-dimethoxyanilin gegenüber dem aus der DE-PS 2 156 051 bekannten Verfahren auf einfachere Weise bei Erzielung gleichguter Ausbeuten und Reinheit hergestellt werden.

Das 4-Chlor-2,5-dimethoxyanilin stellt ein wertvolles Zwischenprodukt zur Herstellung von Farbstoffen und Pigmenten dar.

Die Erfindung wird durch die nachstehenden Beispiele näher erläutert, ohne darauf beschränkt zu werden.

### Beispiel 1

In einem Edelstahlautoklav mit Magnethubvorrichtung, Heizvorrichtung und Kühlung werden

| | |
|---|---|
| 239 g (1,1 mol) | 4-Chlor-2,5-dimethoxy-nitrobenzol, |
| 675 ml | Xylol (technisch), |
| 3 g | 5 % Pt/Kohle, sulfitiert, 50 % Wasser, |
| 1 g | Morpholin, |
| 3 g (0,02 mol) | Dinatriumhydrogenphosphat und |
| 30 ml | Wasser |

vorgelegt.

Nach Verdrängen der Luft im verschlossenen Autoklav mit Stickstoff wird das Reaktionsgemisch auf 85°C erhitzt und unter Rühren Wasserstoff bis auf 10 bar aufgedrückt. Die Reduktion setzt sofort unter Wärmebildung und Abfall des Wasserstoffdruckes ein. Man läßt die Temperatur auf 95°C ansteigen und behält dann diese unter Kühlung bei. Der Druck wird durch weiteres Aufdrücken von Wasserstoff in einem Druckbereich von 5 bis 15 bar gehalten. Wenn kein Druckabfall mehr erfolgt, rührt man noch 30 Minuten bei 95 bis 100°C und einem Druck von 20 bar nach. Die gesamte Reduktionszeit beträgt im Mittel 60 Minuten (gegenüber 75 Minuten beim Verfahren der DE-PS 2 156 051). Nach Entspannen wird der Katalysator bei 95°C unter Stickstoff über ein Druckfilter abgetrennt und in den nächsten Reduktionsansatz zurückgeführt. Das Filtrat wird in einem Rührkolben mit 500 ml Wasser versetzt, das Xylol mit Dampf oder bei schwachem Vakuum (Sumpftemperatur 90 bis 100°C) entfernt und die Base durch Kaltrühren auf 20 bis 25°C als Granulat abgeschieden. Nach Abfiltration fällt ein nahezu farbloses 4-Chlor-2,5-dimethoxyanilin wasserfeucht an, das luftunempfindlich ist und gegebenenfalls noch in üblicher Weise getrocknet werden kann.

Das wäßrige Filtrat zeigt einen pH von 8,2 und das abdestillierte Xylol kann in den nächsten Ansatz zurückgeführt werden. Die Ausbeute beträgt 99 % der Theorie; der EP. (Erstarrungspunkt) liegt bei mindestens 117,8°C, der Diazowert bei ≧ 99 % und der Gehalt an Aminohydrochinondimethylether bei ≦ 0,2 %.

Das gleiche Ergebnis wird erhalten, wenn anstelle von Morpholin Ethylendiamin, Tetramethylethylendiamin oder Di-n-propylamin eingesetzt wird.

Bei Verwendung von Di-n-butylamin oder Tri-n-butylamin wird bei praktisch gleicher Ausbeute mit hellem Aspekt eine geringfügige Erhöhung der Chlorabspaltung (0,1 bis 0,2 %) beobachtet.

### Vergleichsbeispiel 1

Es wurde gemäß Beispiel 1 gearbeitet, jedoch mit dem Unterschied, daß anstelle von sulfitiertem 5 % Pt/Kohle-Katalysator jetzt aktiver 5 % Pt/Kohle-Katalysator eingesetzt wurde. Die Reduktionszeit betrug 53 Minuten. Die Ausbeute lag bei 94,2 % der Theorie, der Gehalt an Aminohydrochinondimethylether bei 1,5 % und der pH des wäßrigen Filtrates bei 2,64. Die isolierte Base zeigte einen blau-violetten Aspekt mit Ep. 115,9°C.

### Vergleichsbeispiel 2

Es wurde gemäß Beispiel 1 gearbeitet, jedoch ohne Zusatz von Morpholin oder eines anderen Amins. Die Reduktionszeit betrug 60 Minuten, die Ausbeute lag bei 99 % der Theorie und der Gehalt an Aminohydrochinondimethylether bei 0,3 bis 0,4 %. Das wäßrige Filtrat zeigte einen pH-wert von 7,8 und der Aspekt der isolierten Base war grau/schwach violett, Ep. 117,3°C.

## Patentansprüche

1. Verfahren zur Herstellung von 4-Chlor-2,5-dimethoxyanilin durch katalytische Reduktion von 4-Chlor-2,5-dimethoxynitrobenzol mit Wasserstoff in flüssiger Phase bei erhöhter Temperatur und erhöhtem Druck, dadurch gekennzeichnet, daß man die Reduktion bei Temperaturen von etwa 80 bis etwa 110°C und einem Druck von etwa 5 bis etwa 50 atü in einem aromatischen Lösungsmittel in Gegenwart eines modifizierten Platin-auf-Kohlenstoff-Katalysators, in Gegenwart von Wasser und von etwa 0,01 bis etwa 0,2 Mol einer Verbindung, die in wäßriger Lösung einen pH-Wert von 8 bis 10 ergibt, und in Gegenwart von etwa 0,1 bis etwa 1,0 Gew.-% eines aliphatischen, offenkettigen primären, sekundären oder tertiären Amins oder eines cyclischen Amins, jeweils bezogen auf das eingesetzte 4-Chlor-2,5-dimethoxynitrobenzol, durchführt.

2. Verfahren nach Anspruch 1, dadurch gekennzeichnet, daß man die Reduktion in Gegenwart eines Amins der allgemeinen Formel 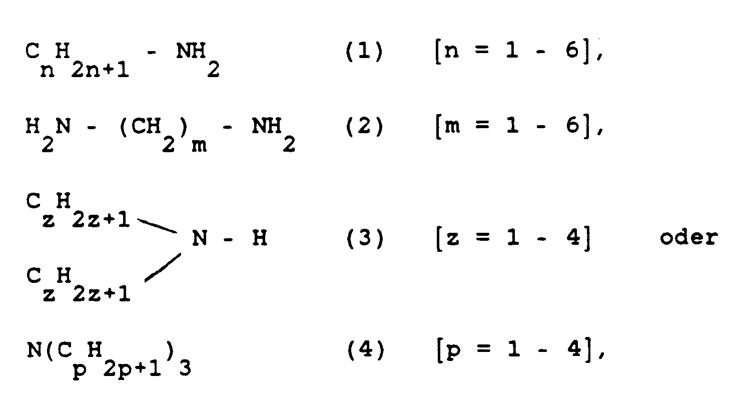 durchführt.

3. Verfahren nach Anspruch 1, dadurch gekennzeichnet, daß man die Reduktion in Gegenwart von Piperidin, Piperazin oder Morpholin durchführt.

4. Verfahren nach mindestens einem der Ansprüche 1 und 2, dadurch gekennzeichnet, daß man die Reduktion in Gegenwart von Tetramethylendiamin durchführt.

5. Verfahren nach mindestens einem der Ansprüche 1 bis 4, dadurch gekennzeichnet, daß man die Reduktion in Gegenwart eines sulfitierten Platin-auf-Kohlenstoff-Katalysators durchführt.

6. Verfahren nach mindestens einem der Ansprüche 1 bis 5, dadurch gekennzeichnet, daß man die Reduktion in Gegenwart von Dinatriumhydrogenphosphat durchführt.

7. Verfahren nach mindestens einem der Ansprüche 1 bis 5, dadurch gekennzeichnet, daß man die Reduktion in Gegenwart von Natriumhydroxid durchführt.

8. Verfahren nach mindestens einem der Ansprüche 1 bis 7, dadurch gekennzeichnet, daß man die Reduktion in Toluol oder einem Xylol oder einem Xylol-Gemisch durchführt.

## Claims

1. A process for the preparation of 4-chloro-2,5-dimethoxyaniline by catalytic reduction of 4-chloro-2,5-dimethoxynitrobenzene with hydrogen in the liquid phase at an elevated temperature and elevated pressure, which comprises carrying out the reduction at temperatures from about 80 to about 110°C and under a pressure of about 5 to about 50 atmospheres gauge in an aromatic solvent in the presence of a modified platinum-on-carbon catalyst in the presence of water and about 0.01 to about 0.2 mole of a compound which gives a pH of 8 to 10 in aqueous solution, and in the presence of about 0.1 to about 1.0 % by weight of an aliphatic, open-chain, primary, secondary or tertiary amine or a cyclic amine, in each case relative to the 4-chloro-2 , 5-dimethoxynitrobenzene employed.

2. The process as claimed in claim 1, wherein the reduction is carried out in the presence of an amine of the formula

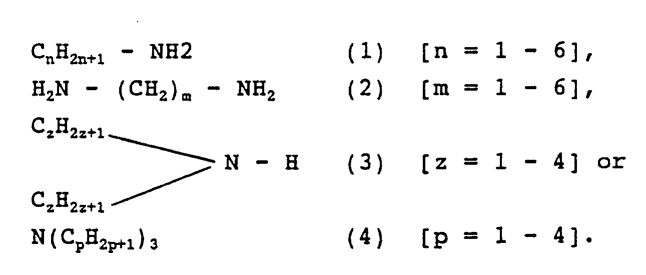

3. The process as claimed in claim 1, wherein the reduction is carried out in the presence of piperidine, piperazine or morpholine.

4. The process as claimed in at least one of claims 1 and 2, wherein the reduction is carried out in the presence of tetramethylenediamine.

5. The process as claimed in at least one of claims 1 to 4, wherein the reduction is carried out in the presence of a sulfited platinum-on-carbon catalyst.

6. The process as claimed in at least one of claims 1 to 5, wherein the reduction is carried out in the presence of disodium hydrogenphosphate.

7. The process as claimed in at least one of claims 1 to 5, wherein the reduction is carried out in the presence of sodium hydroxide.

8. The process as claimed in at least one of claims 1 to 7, wherein the reduction is carried out in toluene or a xylene or a mixture of xylenes.

## Revendications

1. Procédé de préparation de la 4-chloro-,2,5-diméthoxy-aniline par réduction catalytique du 4-chloro-,2,5-diméthoxynitrobenzéne par de l'hydrogène en phase liquide à température élevée et sous pression élevée,caractérisé en ce qu'on effectue la réduction à des températures d'environ 80 à environ 110 °C et sous une surpression d'environ 5 à environ 50 atmosphères (bars) dans un solvant aromatique en présence d'un catalyseur platine sur carbone modifié,en présence d'eau et d'environ0,01 à environ 0,2 mole d'un composé qui donne en solution aqueuse une valeur de pH de 8 à 10, et en présence d'environ 0,1 à environ 1,0 % en poids d'une amine aliphatique primaire, secondaire ou tertiaire en chaîne ouverte ou d'une amine cyclique,le pourcentage étant à chaque fois rapporté au 4-chloro-2,5-diméthoxy-nitrobenzène mis en oeuvre .

2. Procédé selon la revendication 1 , caractérisé en ce qu'on effectue la réduction en présence d'une amine de formule générale :

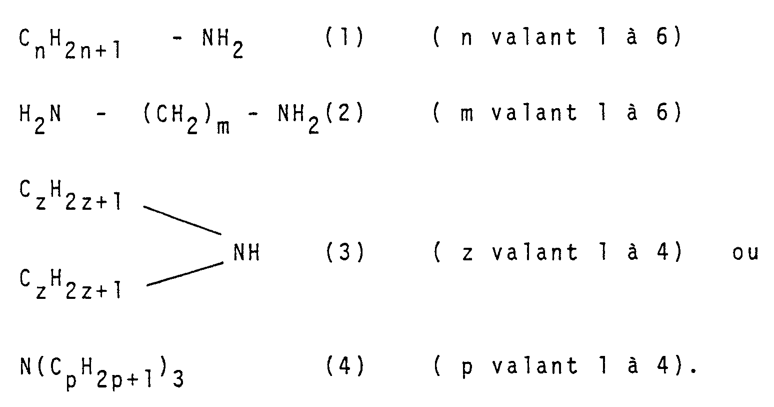

3. Procédé selon la revendication 1 , caractérisé en ce qu'on effectue la réduction en présence de pipéridine, de pipérazine ou de morpholine .

4. Procédé selon l'une au moins des revendications 1 et 2 , caractérisé en ce qu'on effectue la réduction en présence de tétraméthylènediamine .

5. Procédé selon l'une au moins des revendications 1 à 4 , caractérisé en ce qu'on effectue la réduction en présence d'un catalyseur platine-sur-carbone sulfité.

6. Procédé selon l'une au moins des revendications 1 à 5 , caractérisé en ce qu'on effectue la réduction en présence d'hydrogénophosphate disodique.

7. Procédé selon l'une au moins des revendications 1 à 5 , caractérisé en ce qu'on effectue la réduction en présence d'hydroxyde de sodium .

8. Procédé selon l'une au moins des revendications 1 à 7 , caractérisé en ce qu'on effectue la réduction dans du toluène ou dans un xylène ou dans un mélange de xylènes .
